# EUROPEAN PATENT APPLICATION

(11) **EP 2 042 081 A1**
(43) Date of publication of application: **01.04.2009**
(21) Application number: 07117172.2
(22) Date of filing: 25.09.2007
(51) Int. Cl.: A61B 5/00

(54) **Training device for athletes and method for providing information of an athlete**

(71) Applicant: Vodafone Holding GmbH, 40213 Düsseldorf (DE)
(72) Inventor: Gulikers, Harrie, 6213 Maastricht (NL)
(74) Representative: Cullinane, Marietta Bettina

(57) **Abstract**

The present invention relates to a training device (1) for athletes attachable to the body of the athlete, wherein the training device (1) comprises at least one sensor unit (11) for sensing at least one body function and at least one communication unit (151, 153) for communicating with at least one remote processing unit (3). Furthermore a method for providing information of an athlete is described.

## Description

The present invention relates to a training device for athletes as well as to a method for providing information of an athlete.

In the prior art monitoring devices for athletes are known which measure the heart rate of an exerciser during a sports performance. Such a monitoring device is for example described in EP 1 195 135 B1. The monitoring device comprises an electrode belt placed on the chest and a wrist-wore device for processing the received signal and for display of the heart rate. An athlete can thereby monitor his heart rate on the display during exercise.

One drawback of this known device is the limited amount of information which can be made available to the athlete.

The problem underlying the present invention is thus to provide a training device for athletes and a method for providing information which are suitable for supplying at least the athlete with a variety of information.

The invention is based on the finding that this problem can be solved by providing suitable communication means at the training device itself.

According to a first aspect the present invention relates to a training device for athletes attachable to the body of the athlete, wherein the training device comprises at least one sensor unit and at least one communication unit for communicating with at least one remote processing unit.

A training device according to the present invention is a device to be used by an athlete during the performance of exercise. The training device can be a monitoring device, a coaching device, a feed-back device and/or an information provisioning device. The training device is attachable to the body of the athlete, which means that the whole device is carried by the athlete. The sensor unit of the training device may preferably comprise sensors to measure bio signals, such as the heartbeat, pulse, blood pressure and the like. In addition to sensors for bio signals, sensors for measuring environmental conditions such as air pressure, humidity and so on can be used in the training device. The communication unit may comprise a communication module, which preferably includes a SIM card, and an antenna. The SIM card or any other identification module may serve for identifying the training device and thus the athlete towards the remote processing unit. Preferably, the communication unit is connected with the at least one sensor unit via wire connection within the training device. Thereby, the transmission will be secure and negative influences of transmissions via induction can be avoided. The remote processing unit according to the present invention which the training device can communicate with is preferably a network server and will hereinafter also be referred to as a training web server. The communication to such a remote unit is carried out via a communications network, in particular a mobile network such as GSM.

By including a sensor unit and a communication unit in one training device, it will be possible to use any sensor data measured during the exercise. In addition, the usage of only one training device which measures relevant data or signals and is capable of transmitting this data to a remote processing unit is easier and more reliable than the usage of a sensor unit and a separate communication unit which may have to communicate with each other via inductive communication channels such as near field communication. Another advantage of the training device which integrates different units is that the athlete will not be hindered when performing his exercise by having to wear and/or carry several devices. By allowing communication with a remote processing unit it will in particular be possible to obtain information from the processing unit which is not limited in terms of processing power and memory space.

A wrist-worn processing unit which was described in the prior art, in contrast, only provides limited information. The communication with the remote processing unit may be from the training device to the remote processing unit and/or vice versa. With this communication option, data from the training device can be transmitted to the remote processing unit to be processed there, possibly with additional information or data. The thus processed data can be transmitted back to the training device where it can be output. Also information stored on the remote processing unit such as personal profiles, coaching information and so on can be transmitted to the training device to be output. With the training device according to the present invention, it will also be possible to use the data received and the data or information derived from this data at the remote processing unit for a different purpose than retransmission to the athlete. In particular, the data and information can be output to third parties, e.g. can be made available via the Internet for a coach or fans to access.

According to one embodiment, the communication unit comprises a communication module for communicating via a communication network, in particular a mobile network. In addition to being able to communicate with a remote processing unit which may be distant from the athlete, the usage of a communication module for communicating via a communication network also allows for input from other users such as a coach to be transmitted to the training device and to be output there. For example, the coach of the athlete who may be monitoring the exercise via the remote processing unit where the data from the training device is processed and may be displayed, e.g. via the Internet, may contact the athlete to provide coaching instructions.

The training device may further comprise a positioning unit. The positioning unit may comprise an antenna for receiving positioning information from a positioning system such as GPS. By including a positioning unit where the current position and maybe also the speed of the athlete and current altitude can be calculated or received from a remote calculating unit, into the training device, the information which can be provided to the athlete is improved. The improvement being, that the information obtained from the positioning unit can be transmitted to the remote processing unit and can there be processed together with additional information obtained from other sensors of the training device or from profiles stored in the remote processing unit. The profile of the athlete can be identified within the remote processing unit by an identification included in the communication between the training device and the remote processing unit. In particular the SIM card or other identification module may be used to locate the appropriate personal profile at the remote processing unit. Hence, in addition to the sensor data transmitted to the remote processing unit also positioning data can be considered during the processing. For example, if the athlete is currently running on a steep slope, the remote processing unit can identify this circumstance by the positioning data and , if available, additional information stored at the remote processing unit. The sensor data, e.g. the heart rate can thus be evaluated accordingly.

According to a preferred embodiment the training device further comprise an acoustic output interface. This acoustic output interface may for example be a loudspeaker on the training device or a socket for insertion of a headset or an interface for transmitting the audio signals wirelessly to a headset. In either case, the athlete will be provided with information in a non-obtrusive manner. The athlete does not have to focus on a display as required with the prior art device, in order to obtain knowledge about the information of interest. In addition, by using audio signals as output it will be possible to deliver a large variety of information to the user as no limitations such as screen size of the display, have to be observed.

According to another embodiment the training device may further comprises at least one rendering device. The rendering device, in particular for rendering audio content, may be an mp3 player or radio receiver. This embodiment is preferred with a training device having an audio output interface. The inclusion of a rendering device into the training device may be beneficial in various ways. Firstly, the athlete may listen to music or stories while exercising. In addition, since the training device can communicate with a remote processing unit, it will also be possible to include music, stories or cheering slogans into a coaching concept, which will be transmitted to the training device from the remote processing unit. If the remote processing unit identifies for example that the athlete is slowing down but his heart rate is not at a critical value, instructions can be transmitted to the training device to for example play a supporting song or have cheer slogans be output via the rendering device.

The training device according to one embodiment comprises a processing module for processing information received at the training device. This module may in particular be provided to translate or convert received signals into respective output signals. This information may be information relating to the current conditions of the athlete compared to values of a user profile which is stored at the remote processing unit.

Preferably the training device comprises at least one control element for controlling at least the communication unit. The control element may be a button for the user to activate or deactivate the communication unit. Also the status of the communication unit and the selection of settings within the communication channel may be controlled by the control element. For example, an emergency number may be stored in the communication unit which will be dialled if the athlete selects this option. The dialling of the emergency number may for example be effected if the user presses the button three times. Alternatively, a separate emergency button may be provided. It is, however, also possible that the communication unit dials the emergency number upon detection of a crucial sensor data and/or upon receipt of a respective command from the remote processing unit. In this case the control element will be a switch or circuit which is controlled by a processing module of the training device, i.e. without user interaction. The control element may also be used by the user to request specific information. This may be advantageous if the athlete wishes to obtain an intermediate evaluation of his current exercise compared to values laid down in a personal profile at the remote processing unit.

According to a preferred embodiment, the training device is a chest-belt. A chest-belt according to the present invention is a strap or garment which is wrapped tightly around the chest of the athlete. The chest-belt may include a casing for housing units of the training device. This casing is preferably arranged in such a location, that it does not hinder the athlete. The casing may be attached to or included in a strap. One advantage of a chest-belt over a wrist-worn device is that the heart rate can easily be determined. In addition, an output device such as a headset can be attached to the training device without hindering the athlete during his exercise. Furthermore, the length of a chest-belt offers sufficient space to integrate one or more of the described units with the sensors in the same training device.

According to another aspect, the present invention relates to a method for providing information of an athlete. The method is **characterized in that** a training device worn by the athlete and having at least one sensor unit determines sensor data and the sensor data is transmitted from the training device to at least one remote processing unit for further processing. By transmitting the sensor data from the training device where the sensor data is obtained, the transmission of the data can be effected more reliably and faster than with systems where sensor data is transmitted via intermediate communication devices. It will thus be possible to provide real-time transfer of data to the remote processing unit and thus real-time processing of the date. Hence, the athlete or third parties may be provided with information relating to real-time data. The data received and the information processed from this data at the remote processing unit can be provided to the athlete or can be output to third parties, e.g. can be made available via the Internet for a coach or fans to access

According to one embodiment, the training device determines the position of the athlete and transmits the position data to the remote processing unit. The transmission of the position data of the athlete can be performed together or alternating with the transmission of sensor data from the training device to the remote processing unit. By providing the position data from the same training device, the association with received sensor data at the remote processing unit can be facilitated. In particular, a communication unit at the training device may serve to identify the athlete towards the remote processing unit. Sensor data received from this communication unit may then be processed together with position data received from the same communication unit.

The training device may receive the processed data from the remote processing unit and may output the processed data. By receiving processed data or information at the training device, the requirement with respect to processing power and memory of the training device are reduced. As the received, processed data is output from the training device, a separate output device and separate transmission of the data from the remote processing unit to this output device is not necessary. Thereby, the layout of the training device is simplified, as one communication unit will be sufficient. Since the athlete is wearing the training device, the simplified layout of the training device is advantageous as the weight of the training device is minimized.

The processed data and/or information received at the training device is preferably output from the training device as acoustic signals. The output of acoustic signals is beneficial as the output is not limited by specifications such as a screen size or resolution which is the case with visual output signals.

Preferably, the inventive method is carried out with a training device according to the present invention.

The units and modules of the inventive training device may be realized as hardware and/or software components and may at least partially be combined within one unit.

Features and advantages described in context with the inventive training device also apply to the inventive method and vice versa.

The invention will now be described again in detail with reference to the enclosed drawings, wherein:
Figure 1: shows a schematic depiction of an athlete wearing a training device according to the present invention;
Figure 2: shows a schematic view of an embodiment of the inventive training device;
Figure 3: shows a schematic cross sectional view of a sensor unit of the training device;
Figure 4: shows a schematic sectional view of the rear part of the training device according to Figure 2; and
Figure 5: shows a schematic depiction of a system where the inventive training device can be used.

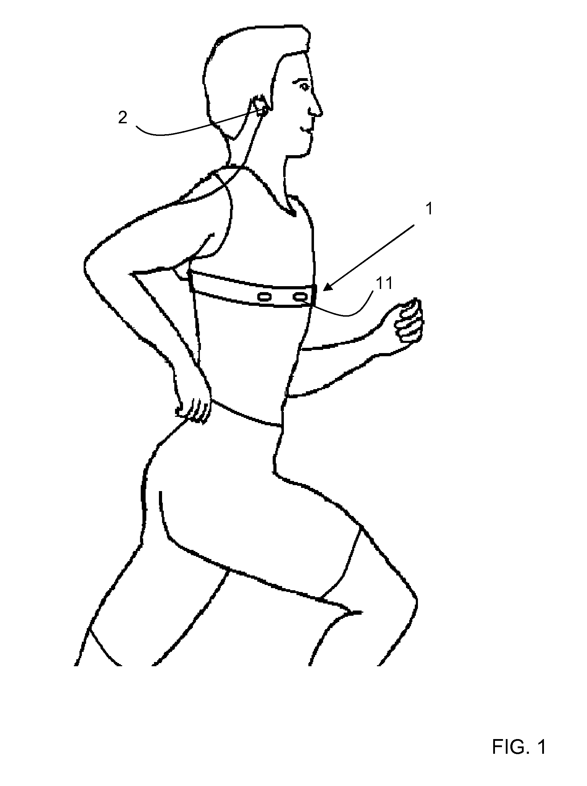

In Figure 1 the training device 1 is shown as a chest-belt worn around the chest of an athlete. On the front of the chest-belt 1 two sensor units 11 are shown, which may for example measure the heart rate of the athlete. Earphones or earplugs 2 are connected to the chest-belt 1 and can be worn by the athlete while the chest-belt 1 is placed around his chest.

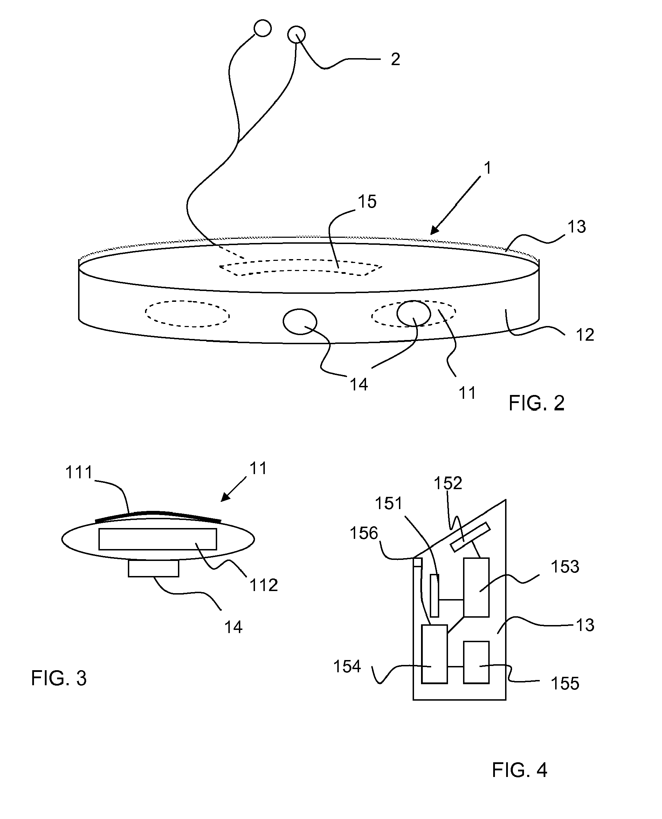

As shown in Figure 2, the chest-belt 1 comprises a strap 12, which forms the front part of the chest-belt 1 as well as a casing 13 which forms the back part of the chest-belt 1. Sensor units 11 are arranged on or in the strap 12. Furthermore touch buttons 14 are included in the strap 12. One touch button 14 is shown in Figure 2 to be spaced from the sensor unit 11, whereas another touch button 14 is included in the sensor unit 11.

In Figure 3 the layout of a sensor unit 11 is schematically shown. The sensor unit 11 comprises a sensor 111, which may be a heart rate pad; furthermore a battery 112 is included in the sensor unit 11. The battery 112 may be made from a Li-Polymer, as these can easily be formed to fit in the chest-belt 1. The sensor units 11 are mini sensor units so that they do not cause discomfort for the athlete. At the front of the sensor unit 11 the touch button 14 is arranged. The sensor 111 as well as the battery 112 and the touch button 14 are connected to further units of the training device, in particular to a central unit 15, which in Figure 2 is shown to be arranged within the back part 13 of the chest-belt.

The central unit 15 is schematically shown in Figure 4. In the depicted embodiment, the central unit 15 comprises a GSM antenna 151, a GPS antenna 152, a communication module 153 as well as a processing module 154. Furthermore a rendering device 155 is included in the central unit 15. Finally, an output interface 156 is provided.

The GSM antenna 151 is adapted to communicate with the communication module 153. Thereby signals received at the GSM antenna 151 can be forwarded to the communication module 153 and signals generated within the central unit 15 can be transmitted to the GSM antenna 151 for further transmission. The GSM antenna 151 is arranged in the central unit 15 such that it is located at the side of the chest-belt, which will be facing away from the athlete when worn.

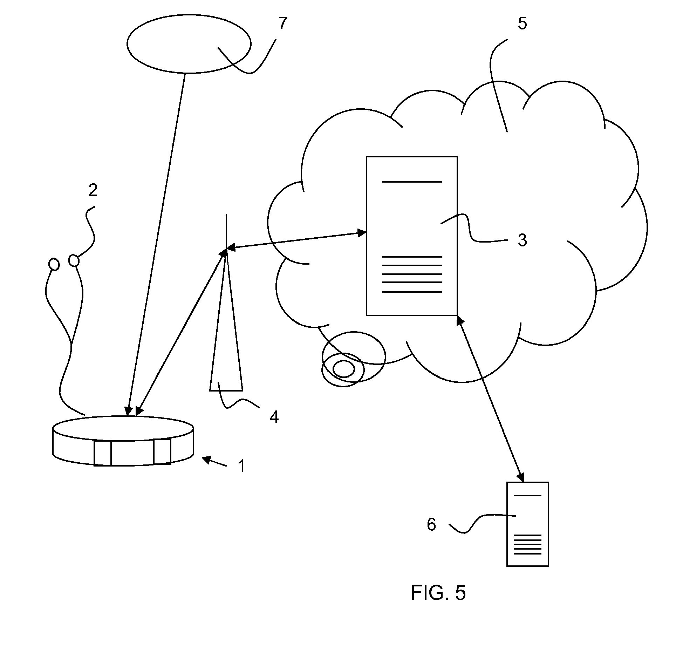

Also the GPS antenna 152 is connected to the communication module 153, so that positioning data can be obtained at the communication module 153. The GPS antenna 152 is preferably arranged within the training device 1 such that it is directed towards the sky, but slightly away from the head of the athlete. In Figure 4 the GPS antenna 152 is thus arranged at an angle to the horizontal plane at the top of the central unit 15. The communication module 153 may be a GPS/GSM module, preferably a mini module, with a SIM card and may be adapted to calculate the current position, speed and altitude of the athlete. In addition, real-time communication with a remote processing unit 3 (Figure 5) can be realized. The calculation of the position, speed and altitude of the athlete may also be performed in the processing unit 154 of the training device 1 or in the remote processing unit 3.

The processing unit 154 is connected to the communication module 153 as well as to the rendering device 155. Finally, the processing unit 154 is also connected to the output interface 156. The output interface 156 may be a socket for receiving a plug of earphones or headsets 2. It should be noted that the arrangement of the units and modules in Figure 4 is only schematic and may differ from the depicted embodiment.

In Figure 5 the training device 1 is shown in connection with the system wherein it can preferably be operated. The GSM antenna 151 allows for communication of the training device 1 to a remote processing unit 3, in particular a network server 3, via a mobile communication network, which is depicted in Figure 5 by a base station 4, and the Internet 5. The network server 3 may also be accessed by a different terminal device, such as a personal computer PC 6 or mobile phone. The position data which will be received at the GPS antenna 152 will be provided by a positioning system 7 such as a GPS system.

The working of the training device 1 will now be described with reference to the Figures.

When the athlete starts his exercise, he can place the chest-belt 1 around his chest. It will be positioned such that at least one sensor unit 11 is located in the vicinity of the heart of the athlete. While exercising, the heart rate of the athlete will be measured by one of the sensor units 11. The sensor data will be transmitted in real time to the central processing unit 3, where further processing will be carried out. At the central processing unit 3, for example, a personal profile of the athlete may be stored. The personal profile may include bio data such as the maximum allowable heart rate for the athlete. Additionally or alternatively, the personal profile may comprise information relating to training or coaching schedules for the specific athlete. Also pre-selected routes for an exercise may be stored in the central processing unit 3.

The sensor units 11 may also detect other data than the heart rate. For example, the loss of fluid or other values can be measured. In addition to sensor data also positioning data may be transmitted to the central processing unit 3. The positioning data will be obtained by the GPS system 7 and received at the training device via the GPS antenna 152. The position data can be processed at the remote processing unit 3 together with the received sensor data. In this case, the processed data may result in an instruction signal to be output to the athlete that his speed in view of his heart rate is too high. Alternatively, the position data may be compared to stored information such as a map of a preferred route and respective directional instruction signals may be transmitted to the training device 1 to be output to the athlete.

The signals received at the training device 1 via the GSM antenna 151 may be audio signals which can directly be output to the athlete. Alternatively, the signals may be processed at the processing unit 154 to generate an audio output signal. The audio signal will be output via the audio output interface 156. In the depicted embodiment earplugs 2 are attached to the audio output interface 156. Thereby the athlete can receive the real time feed back without being disturbed in his exercise.

During the exercise, also entitled third parties may access the data and/or the personal profile of the athlete on the remote processing unit 3. This can be carried out via a PC 6 and using the Internet 5. Input from the third party such as coaching instructions from a coach can be transmitted from the remote processing unit 3 to the training device 1 and can be output to the athlete.

If the athlete wishes to interrupt the feed back from the central processing unit 3 and/or the transmission of data to the central processing unit 3, one of the touch buttons 14 may be pressed, whereby the communication module 153 cancels the communication with the remote processing unit 3.

The touch buttons 14 may also be adapted to provide the athlete with the ability to call a predefined emergency number. By pushing the button 14, the communication module 153, where also the number may be stored, will establish a connection to this number via the GSM antenna 151.

Another button 14 may be connected to the rendering device 155 so that the athlete can start and stop rendering of music or other sound by touching the button 14.

As the buttons 14 are preferably arranged at the front of the training device 1 they are easily accessible for the athlete during his exercise.

The inventive device 1 allows for real-time acoustic feedback about the current status versus training objectives. The feed back can be given via predefined rules which are stored in the central processing unit 3, in particular in a training web server. Alternatively, the feedback may be provided by a real coach using a PC or a mobile phone to provide the information to the athlete. The input from a real coach may be provided via the central processing unit 3 or directly via the GSM antenna 151 of the training device 1.

As all units are included in one training device 1, the comfort of the athlete wearing the training device 1 is increased. In particular all required sensors are included in the training device 1.

With the communication unit 153 being provided within the training device 1, it will also be possible to share and download favourite tracks via or from the remote processing unit 3. It is also possible to download performances of other athletes from the remote processing unit 3 to the training device 1 and to thereby train against or compete with the other athlete. Instead of downloading the performance date to the training device 1, it is also possible, that data received at the central processing unit 3, such as sensor data and speed or location data is compared at the central processing unit 3 against the performance data of other athletes. The performance data of the athlete may be stored in the training device 1 or on the remote processing unit 3.

As also a rendering device 155 is preferably provided in the training device 1, the athlete will be provided with the ability to listens to his favourite song during the exercise without having to carry or wear a separate rendering device. Another advantage of including a rendering device 155 within the training device 1 is that the rendering of music can be interrupted if coaching instructions or other feed back from the central processing unit 3 is received at the training device 1.

With the present invention an all-in-one device is provided for an athlete to be used during his exercise.

### Reference Numbers

- 1: training device / chest belt
- 11: sensor unit
- 111: sensor
- 12: strap
- 13: casing
- 14: touch button
- 15: central unit
- 151: GSM antenna
- 152: GPS antenna
- 153: communication module
- 154: processing unit
- 155: rendering device
- 156: output interface
- 2: earplugs
- 3: network server
- 4: base station
- 5: Internet
- 6: PC
- 7: GPS system

## Claims

1. Training device for athletes attachable to the body of the athlete, wherein the training device (1) comprises at least one sensor unit (11) for sensing at least one body function and at least one communication unit (151, 153) for communicating with at least one remote processing unit (3).

2. Training device according to claim 1, **characterized in that** the communication unit (151, 153) comprises a communication module (153) for communicating via a communication network (4), in particular a mobile radio network.

3. Training device according to anyone of claims 1 or 2, **characterized in that** the training device (1) further comprises a positioning unit (152, 153).

4. Training device according to anyone of claims 1 to 3, **characterized in that** the training device (1) further comprises an acoustic output interface (156).

5. Training device according to anyone of claims 1 to 4, **characterized in that** the training device (1) further comprises at least one rendering device (155).

6. Training device according to anyone of claims 1 to 5, **characterized in that** the training device (1) comprises a processing module (154) for processing information received at the training device (1).

7. Training device according to anyone of claims 1 to 6, **characterized in that** the training device (1) comprises at least one control element (14) for controlling at least the communication unit (151, 153).

8. Training device according to anyone of claims 1 to 7, **characterized in that** it is a chest-belt.

9. Method for providing information of an athlete, **characterized in that** a training device (1) worn by the athlete and having at least one sensor unit (11) determines sensor data and the sensor data is transmitted by the training device (1) to at least one remote processing unit (3) for further processing.

10. Method according to claim 9, **characterized in that** the training device (1) detects the position of the athlete and transmits the position data to the remote processing unit (3).

11. Method according to claim 9 or 10, **characterized in that** the training device (1) receives the processed data from the remote processing unit (3) and generates an output signal according to the processed data.

12. Method according to anyone of claims 9 to 11, **characterized in that** the processed data and/or information received at the training device (1) is output from the training device (1) as acoustic signals.

13. Method according to anyone of claims 9 to 12, **characterized in that** the method is carried out with a training device (1) according to anyone of claims 1 to 8.
